# EUROPÄISCHE PATENTSCHRIFT

(11) **EP 0 758 334 B1**
(45) Veröffentlichungstag und Bekanntmachung des Hinweises auf die Patenterteilung: **05.02.2003**
(21) Anmeldenummer: 95916679.4
(22) Anmeldetag: 20.04.1995
(51) Int. Cl.: C07D 491/056, A01N 43/90

(54) **SUBSTITUIERTE (1,4)DIOXINO(2,3-F)BENZIMIDAZOLDERIVATE UND IHRE VERWENDUNG ZUR BEKÄMPFUNG VON SCHÄDLINGEN**
SUBSTITUTED (1,4)DIOXINO(2,3-F)BENZOIMIDAZOLE DERIVATIVES AND THEIR USE IN PEST CONTROL
DERIVES DE (1,4)DIOXINO(2,3-F)BENZIMIDAZOLE SUBSTITUES ET LEUR UTILISATION DANS LA LUTTE CONTRE LES PARASITES

(30) Priorität: 03.05.1994 DE 4415435
(43) Veröffentlichungstag der Anmeldung: 19.02.1997
(73) Patentinhaber: Bayer CropScience AG, 40789 Monheim (DE)
(72) Erfinder: ASSMANN, Lutz, D-23701 Eutin (DE); MARHOLD, Albrecht, D-51373 Leverkusen (DE); DEHNE, Heinz-Wilhelm, D-53125 Bonn (DE); HÄNSSLER, Gerd, D-51381 Leverkusen (DE); STENZEL, Klaus, D-40595 Düsseldorf (DE); WACHENDORFF-NEUMANN, Ulrike, D-56566 Neuwied (DE)
(86) Internationale Anmeldenummer: EP9501500
(87) Internationale Veröffentlichungsnummer: WO95029918

(56) Entgegenhaltungen:
- EP-A- 0 517 476
- EP-A- 0 545 204
- EP-A- 0 630 570
- DE-A- 4 237 567

## Beschreibung

Die Erfindung betrifft Benzimidazol-Derivate, ein Verfahren zu ihrer Herstellung und ihre Verwendung als Schädlingsbekämpfungsmittel.

Es ist bekannt, dass bestimmte Benzimidazol-Derivate, wie beispielsweise das 2-Cyano-3-dimethylaminosulfonyl-6,6,7,7-tetrafluor-[1,4] dioxino [2,3-f] benzimidazol oder das 2-Cyano-6,6-difluor-2-dimethylaminosulfonyl-[1,3]dioxolo [4,5-f] benzimidazol fungizide bzw. auch akarizide Eigenschaften aufweisen (vgl. EP-A 0 517 476, EP-A 0 545 204 bzw. DE-A 4 139 950). Die Wirksamkeit dieser vorbekannten Verbindungen ist jedoch, insbesondere bei niedrigen Aufwandmengen nicht in allen Anwendungsgebieten völlig zufriedenstellend. Außerdem werden in den genannten Druckschriften keine Stoffe offenbart, in denen sowohl ein tetrahalogenierter Dioxino-Teil als auch eine Alkyl-SO₂-Gruppe vorhanden ist.

Schließlich betrifft die EP-A 0 630 570 synergistisch wirksame, fungizide Mittel, in denen unter anderem das 2-Cyano-3-dimethylsulfonyl-6,6,7,7-tetrafluor-[1,4]-dioxino[2,3-f]benzimidazol enthalten ist. Entsprechende Verbindungen, die einen Alkyl-SO₂-Rest als Substituenten am Imidazol-Ring aufweisen, werden aber nicht erwähnt.

Es wurden nun Benzimidazol-Derivate der Formel 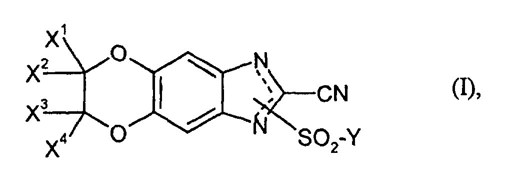 in welcher
- X¹ und X³: für Fluor stehen,
- X² und X⁴: für Chlor stehen und
- Y: für Methyl steht,
oder
- X¹, X², X³ und X⁴: für Fluor stehen und
- Y: für Methyl, Ethyl, 3-Chlor-propyl, i-Butenyl oder Benzyl steht
gefunden.

In der Formel (I) steht die gestrichelte Linie für eine Doppelbindung zwischen einem der beiden Stickstoffatome und dem benachbarten Kohlenstoffatom, das den Substituenten CN trägt. Das Stickstoffatom, das nicht an der Doppelbindung beteiligt ist, trägt jeweils den -SO₂-Y Rest.

Weiterhin wurde gefunden, dass man Benzimidazol-Derivate der Formel (I) erhält, wenn man Benzimidazole der Formel 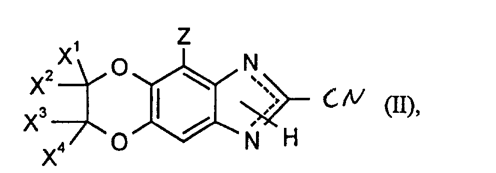 in welcher
- X¹, X², X³ und X⁴: die oben angegebene Bedeutung haben,
mit Sulfonylchloriden der Formel

Cl-SO₂-Y (III),

in welcher
- Y: die oben angegebene Bedeutung hat,
gegebenenfalls in Gegenwart eines Verdünnungsmittels und gegebenenfalls in Gegenwart eines Reaktionshilfsmittels umsetzt.

Weiterhin wurde gefunden, dass Benzimidazol-Derivate der Formel (I) sehr gut als Schädlingsbekämpfungsmittel geeignet sind. Sie zeichnen sich insbesondere durch hohe fungizide, insektizide und akarizide Wirksamkeit aus.

Überraschenderweise zeigen die erfindungsgemäßen Benzimidazol-Derivate eine bessere Wirksamkeit als die konstitutionell ähnlichsten vorbekannten Verbindungen.

Verwendet man beispielsweise 2-Cyano-6,6,7,7-tetrafluor-[1,4] dioxino [2,3-f] benzimidazol und Methansulfonsäurechlorid als Ausgangsstoffe, so lässt sich der Reaktionsablauf des erfindungsgemäßen Verfahrens durch das folgende Formelschema darstellen:

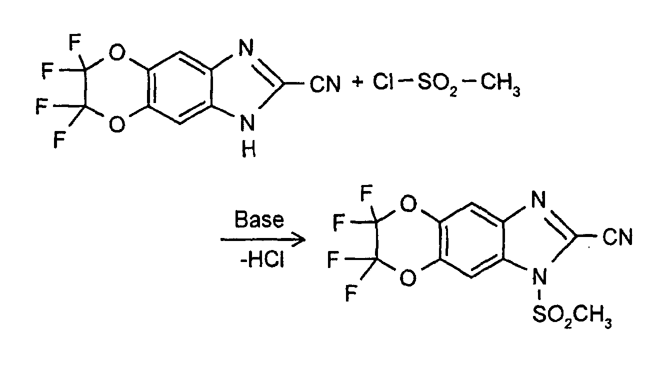

Die Benzimidazole der Formel (II) sind teilweise bekannt (vgl. EP-A 0 517 476 oder DE-OS 4 139 950). Sie lassen sich herstellen, indem man Phenylendiamine der Formel 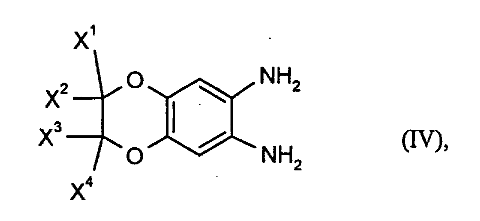 in welcher
- X¹, X², X³ und X⁴: die oben angegebenen Bedeutungen haben,
mit 2,2,2-Trichloracetimidsäuremethylester der Formel 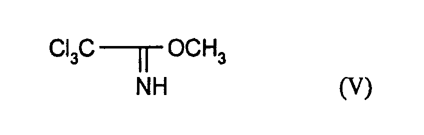 in Gegenwart von Eisessig bei Temperaturen zwischen 10°C und 40°C umsetzt und die dabei entstehenden 2-Trichlormethyl-benzimidazole der Formel 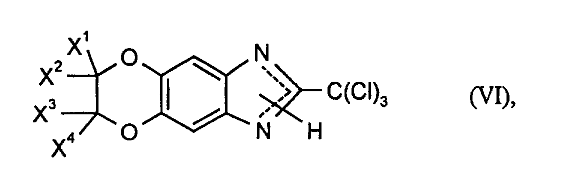 in welcher
- X¹, X², X³ und X⁴: die oben angegebenen Bedeutungen haben,
mit wässriger Ammoniak-Lösung in Gegenwart eines Verdünnungsmittels, wie z.B. Ethanol, bei Temperaturen zwischen 0°C und 40°C umsetzt.

Die Verbindungen der Formeln 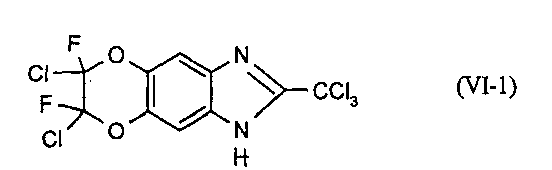 und 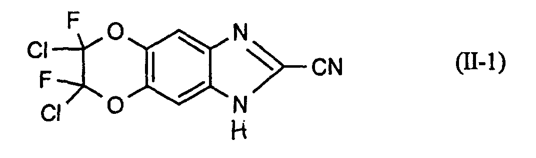 sind neu. Sie lassen sich nach dem obigen Verfahren herstellen, indem man 6,7-Diamino-2,3-dichlor-2,3-difluor-[1,4]-benzodioxan der Formel 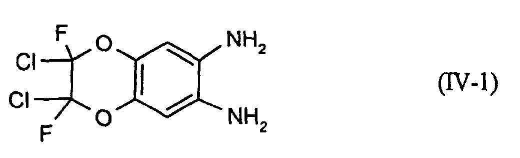 mit 2,2,2-Trichloracetimidsäuremethylester der Formel 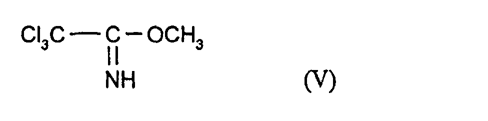 in Gegenwart von Eisessig bei Temperaturen zwischen 10°C und 40°C umsetzt und das dabei entstehende 6,7-Dichlor-6,7-difluor-2-trichlormethyl-[1,4]-dioxino-[2,3-f]-benzimidazol der Formel 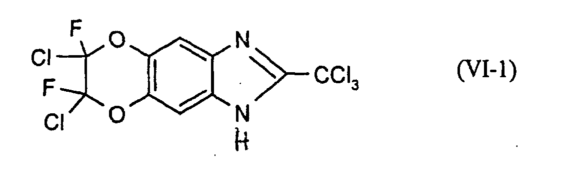 mit wässriger Ammoniak-Lösung in Gegenwart eines Verdünnungsmittels, wie z.B. Ethanol, bei Temperaturen zwischen 0°C und 40°C umsetzt.

Die Phenylendiamine der Formel (IV) sind teilweise bekannt (vgl. EP-A 0 517 476, DE-OS 4 139 950, DE-OS 3 621 301 und DE-OS 3 605 977). Sie lassen sich herstellen, indem man 1,4-Benzodioxane der Formel 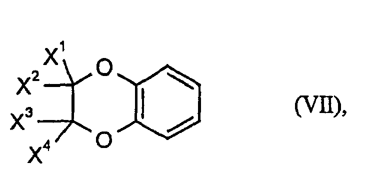 in welcher
- X¹, X², X³ und X⁴: die oben angegebenen Bedeutungen haben,
mit einem Gemisch aus Salpetersäure und Schwefelsäure (das auch Oleum enthalten kann) bei Temperaturen zwischen 0°C und 100°C umsetzt und die so erhaltenen Dinitro-1,4-benzodioxane der Formel 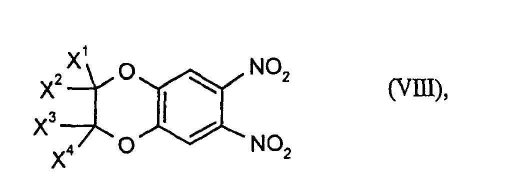 in welcher
- X¹, X², X³ und X⁴: die oben angegebenen Bedeutungen haben,
entweder z.B. mit Eisen in Gegenwart von wässriger Salzsäure und Ethanol bei Temperaturen zwischen 50°C und 100°C reduziert,
oder katalytisch mit elementarem Wasserstoff bei Temperaturen zwischen 25°C und 100°C und unter einem Druck zwischen 1 and 100 bar sowie in Gegenwart von Metallen oder Metallverbindungen der VIII. Nebengruppe des Periodensystems der Elemente, insbesondere Nickel oder Palladium als Katalysatoren (vgl. auch die Herstellungsbeispiele) umsetzt.

Bisher noch nicht bekannt ist das 6,7-Diamino-2,3-dichlor-2,3-difluor-[1,4]-benzodioxan der Formel

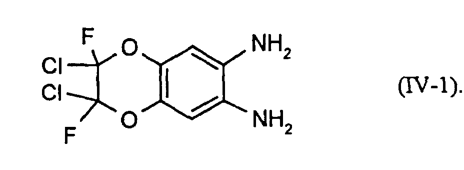

Es lässt sich nach dem obigen Verfahren herstellen, indem man 2,3-Dichlor-2,3-difluor-6,7-dinitro[1,4]-benzodioxan der Formel 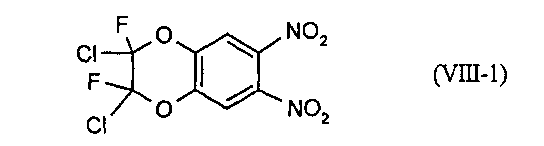 entweder z.B. mit Eisen in Gegenwart von wässriger Salzsäure und Ethanol bei Temperaturen zwischen 50°C und 100°C reduziert,
oder katalytisch mit elementarem Wasserstoff bei Temperaturen zwischen 25°C und 100°C und unter einem Druck zwischen 1 und 100 bar sowie in Gegenwart von Metallen oder Metallverbindungen der VIII. Nebengruppe des Periodensystems der Elemente, insbesondere Nickel oder Palladium als Katalysatoren (vgl. auch die Herstellungsbeispiele) umsetzt.

Bisher noch nicht bekannt ist auch das 2,3-Dichlor-2,3-difluor-6,7-dinitro-[1,4]-benzodioxan der Formel

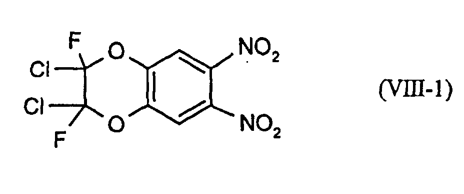

Die Verbindung der Formel (VIII-1) lässt sich herstellen, indem man 2,3-Dichlor-2,3-difluor-[1,4]-benzodioxan der Formel 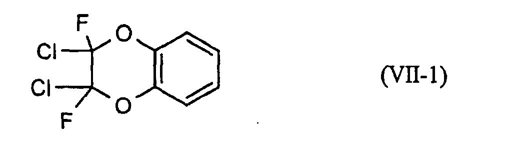 mit einem Gemisch aus Salpetersäure und Schwefelsäure, gegebenenfalls in Gegenwart von Oleum, bei Temperaturen zwischen 0°C und 100°C umsetzt.

Das 2,3-Dichlor-2,3-difluor-[1,4]-benzodioxan der Formel (VII-1) ist bisher noch nicht bekannt. Die Verbindung lässt sich herstellen, indem man 2,2,3,3-Tetrachlor-[1,4]-benzodioxan der Formel 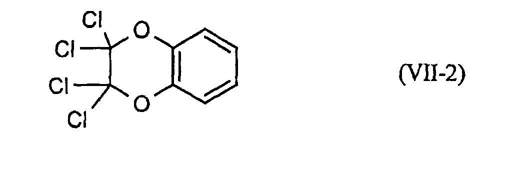 mit wasserfreier Flusssäure im Autoklaven bei Temperaturen zwischen -10°C und +70°C umsetzt (vgl. Herstellungsbeispiele).

Als Verdünnungsmittel zur Durchführung des erfindungsgemäßen Verfahrens kommen inerte organische Lösungsmittel infrage. Vorzugsweise verwendbar sind aliphatische, alicyclische oder aromatische, gegebenenfalls halogenierte Kohlenwasserstoffe wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether wie Diethylether, Diisopropylether, Dioxan, Tetrahydrofuran oder Ethylenglykoldimethyloder -diethylether, Ketone wie Aceton oder Butanon oder Methyl-isobutyl-keton; Nitrile wie Acetonitril, Propionitril oder Benzonitril oder Ester wie Essigsäuremethylester oder Essigsäureethylester.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Vorzugsweise verwendbar sind Erdalkalimetall- oder Alkalimetallhydroxide wie Natriumhydroxid, Calciumhydroxid, Kaliumhydroxid oder auch Ammoniumhydroxid, Alkalimetallcarbonate wie Natriumcarbonat, Kaliumcarbonat, Kaliumhydrogencarbonat, Natriumhydrogencarbonat, Alkali- oder Erdalkalimetallacetate wie Natriumacetat, Kaliumacetat oder Calciumacetat sowie tertiäre Amine wie Trimethylamin, Triethylamin, Tributylamin, N,N-Dimethylanilin, Pyridin, N-methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise bei Temperaturen zwischen 20°C und 120°C.

Das erfindungsgemäße Verfahren wird üblicherweise unter Normaldruck durchgeführt. Es ist jedoch auch möglich, unter erhöhtem oder vermindertem Druck zu arbeiten.

Zur Durchführung des erfindungsgemäßen Verfahrens setzt man pro Mol an Benzimidazol der Formel (II) in einem Verdünnungsmittel im allgemeinen 1,0 bis 2,0 Mol, vorzugsweise 1,0 bis 1,3 Mol an Sulfonylchlorid der Formel (III) und gegebenenfalls 1,0 bis 2,0 Mol, vorzugsweise 1,0 bis 1,3 Mol an Reaktionshilfsmittel ein. Die Reaktionsführung, Aufarbeitung und Isolierung der Reaktionsprodukte erfolgt nach üblichen Methoden (vgl. auch die Herstellungsbeispiele).

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide werden im Pflanzenschutz eingesetzt zur Bekämpfung von Plasmodiophoromycetes, Oomycetes, Chytridiomycetes, Zygomycetes, Ascomycetes, Basidiomycetes und Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus
(Konidienform: Drechlera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellucularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Krankheiten im Obst- und Gemüsebau, wie beispielsweise gegen den Erreger der Tomatenbraunfäule (Phytophthora infestans) oder gegen den Erreger des Apfelschorfs (Venturia inaequalis) oder gegen den Erreger des falschen Mehltaus an Reben (Plasmopara viticola) oder zur Bekämpfung von Reis-Krankheiten, wie beispielsweise gegen den Erreger der Reisfleckenkrankheit (Pyricularia oryzae) eingesetzt werden.

Außerdem zeigen die erfindungsgemäßen Wirkstoffe eine gute in-vitro-Wirkung.

Darüber hinaus eignen sich die erfindungsgemäßen Wirkstoffe zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofinannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Wirkstoffe der Formel (I) zeichnen sich auch durch hervorragende akarizide Wirkung aus, z.B. gegen die gemeine Spinnmilbe (Tetranychus urticae). Sie zeigen aber auch eine gute blattinsektizide Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächen-aktiven Mitteln. Im Falle der Benutzung von Wasser als Streckmittel können z.B. auch organische Lösungsmittel als Hilfslösungsmittel verwendet werden.

Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlen-wasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokusnussschalen, Maiskolben und Tabakstengel; als Emulgier- und/oder schaumerzeugende Mittel kommen in Frage; z.B. nichtionogene ond anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexföromige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetable Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Fungizide als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Besonders günstige Mischpartner sind z.B. die folgenden Verbindungen:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoximino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-otolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin,
Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat,
Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol,
Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos,
Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können bei der Anwendung als Fungizide als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.

Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen verhandelt werden.

Bei der Behandlung von Pflanzenteilen können die Wirkstoffkonzentrationen in den Anwendungsformen bei der Anwendung als Fungizide in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden bei der Anwendung als Fungizide im allgemeinen Wirkstoffmengen von 0,001 bis 50 g je Kilogramm Saatgut, vorzugsweise 0,01 bis 10g benötigt.

Bei Behandlung des Bodens sind bei der Anwendung als Fungizide Wirkstoffkonzentrationen von 0,00001 bis 0,1 Gew.-%, vorzugsweise von 0,0001 bis 0,02 % am Wirkungsort erforderlich.

Die erfindungsgemäßen Wirkstoffe können bei der Anwendung als Insektizide und Akarizide in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Genannt seien die folgenden Verbindungen:
Acrinathrin, Alphamethrin, Betacyfluthrin, Bifenthrin, Brofenprox, Cis-Resmethrin,
Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrirriphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion,
Buprofezin, Chlorfluazuron, Diflubenzuron, Flucycloxuron, Flufenoxuron, Hexaflumuron, Pyriproxifen, Tebufenozide, Teflubenzuron, Triflumuron,

Imidacloprid, Nitenpyram, N-[(6-Chloro-3-pyridinyl)methyl]-N'-cyano-N-methylethanimidamid (NI-25),
Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril (AC 303630).

Die erfindungsgemäßen Wirkstoffe können bei der Anwendung als Insektizide und Akarizide ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv sein muss.

Der Wirkstoffgehalt der aus den handelsüblichen Formulierungen bereiteten Anwendungsformen kann bei der Anwendung als Insektizide und Akarizide in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Die Herstellung und die Verwendung der erfindungsgemäßen Wirkstoffe gehen aus den nachfolgenden Beispielen hervor.

### Herstellungsbeispiele

### Beispiel 1

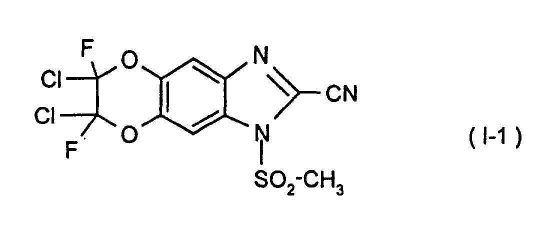

Ein Gemisch aus 0,6 g (15 mmol) 60 %-igem Natriumhydrid in 70 ml Tetrahydrofuran wird bei Raumtemperatur unter Rühren portionsweise mit 4,6 g (15 mmol) 2-Cyano-6,7-dichlor-6,7-difluor-[1,4]dioxino [2,3-f]benzimidazol versetzt. Man rührt noch 30 Minuten bei Raumtemperatur, gibt dann 4,6 g (30 mmol) Methansulfonsäurechlorid hinzu und erhitzt 12 Stunden unter Rückfluss.

Nach dem Abkühlen wird die Lösung auf 400 ml Wasser gegossen. Man extrahiert dreimal mit je 100 ml Methylenchlorid extrahiert und engt die vereinigten organischen Extrakte nach dem Trocknen unter vermindertem Druck ein. Anschließend wird der erhaltene Rückstand zur Kristallisation mit 20 ml Ether verrrührt.

Man erhält 4,9 g (85 % der Theorie) 2-Cyano-6,7-dichlor-6,7-difluor-3-methylsulfonyl-[1,4]dioxino [2,3-f] benzimidazol vom Schmelzpunkt 123 bis 126°C.

### Herstellung der Ausgangsprodukte

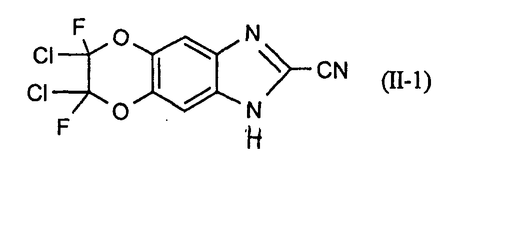

Zu einer Mischung von 250 ml konzentrierter, wässriger Ammoniak-Lösung und 200 ml Ethanol werden unter Rühren bei Raumtemperatur 59,7 g (0,15 Mol) 6,7-Dichlor-6,7-difluor-2-trichlormethyl-[1,4] dioxino [2,3-f] benzimidazol gegeben. Das Gemisch wird 20 Stunden bei Raumtemperatur gerührt und dann mit Salzsäure angesäuert. Das Reaktionsgemisch wird dreimal mit je 150 ml Methylenchlorid extrahiert. Die organischen Extrakte werden getrocknet und eingeengt. Der erhaltene Rückstand wird dann auf Kieselgel mit Diethylether als Laufmittel chromatographisch gereinigt.

Man erhält 23,8 g (52 % der Theorie) 2-Cyano-6,7-dichlor-6,7-difluor-[1,4] dioxino [2,3-f] benzimidazol vom Schmelzpunkt >230°C.

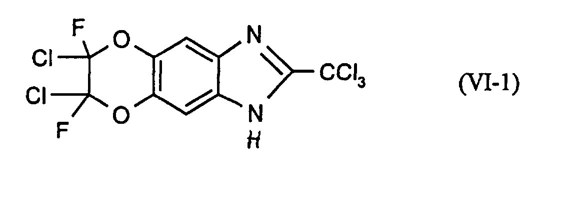

In ein Gemisch aus 54,2 g (0,20 Mol) 6,7-Diamino-2,3-dichlor-2,3-difluor-[1,4]-benzodioxan und 200 ml Eisessig werden bei Raumtemperatur unter Rühren 54,0 g (0,3 Mol) 2,2,2-Trichloracetimidsäuremethylester zugetropft. Nach beendeter Zugabe wird noch 20 Stunden bei Raumtemperatur gerührt. Dann werden 300 ml Wasser zugegeben, und der erhaltene Niederschlag wird abfiltriert und getrocknet.

Man erhält 63,1 g (79 % der Theorie) 6,7-Dichlor-6,7-difluor-2-trichlormethyl-[1,4] dioxino [2,3-f] benzimidazol vom Schmelzpunkt >230°C.

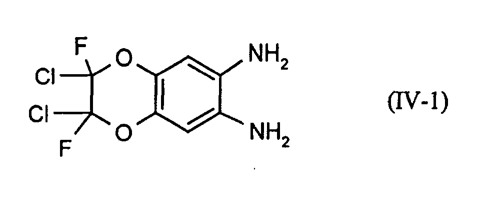

In einem Hydrierautoklaven werden 316 g (0,95 Mol) 2,3-Dichlor-2,3-difluor-6,7-dinitro-[1,4]-benzodioxan in 1500 ml Tetrahydrofuran zusammen mit 30 g Raney-Nickel vorgelegt, mit Wasserstoff gespült und anschließend durch Aufdrücken von 30 bis 50 bar Wasserstoff bei 25 bis 45°C hydriert. Wenn kein Wasserstoff mehr absorbiert wird, wird abgekühlt und entspannt. Nach dem Abfiltrieren des Katalysators wird das Lösungsmittel abdestilliert.

Man erhält 246 g 6,7-Diamino-2,3-dichlor-2,3-difluor-[1,4]-benzodioxan vom Schmelzpunkt 130 bis 131°C.

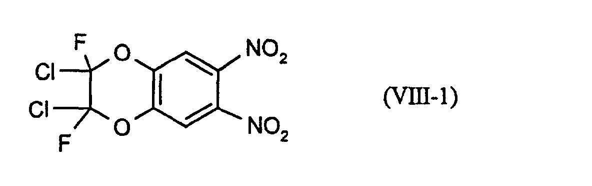

In einer Rührapparatur werden 400 g Mischsäure (33 Gew.-% HNO₃, 67 Gew.-% H₂SO₄) bei 20°C vorgelegt und 200 g 2,3-Dichlor-2,3-difluor-1,4-benzodioxan unter Rühren bei Raumtemperatur zugetropft. Man rührt 1 Stunde bei 20°C und 1 Stunde bei 40°C nach. Dann werden 100 ml 20 %-iges Oleum zugetropft. Es wird 2 Stunden bei 40°C und dann 3 Stunden bei 50°C nachgerührt. Nach dem Abkühlen wird das Gemisch auf 500 g Eis ausgetragen und das Produkt in Dichlormethan aufgenommen. Nach dem Waschen mit Wasser wird die Lösung getrocknet und anschließend vom Lösungsmittel befreit.

Man erhält 245 g 2,3-Dichlor-2,3-difluor-6,7-dinitro-[1,4]-benzodioxan.

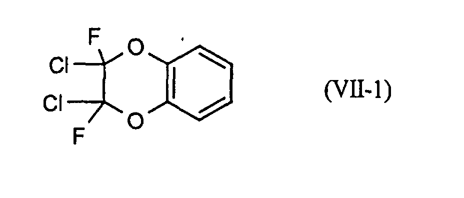

In einer Fluorierungsapparatur werden 1800 ml wasserfreier Fluorwasserstoff vorgelegt und 1300 g 2,2,3,3-Tetrachlor-[1,4]-benzodioxan bei -10°C eingetragen. Dann wird der Autoklav verschlossen und der Inhalt unter Rühren auf 60°C erhitzt. Nach dem Ende der Chlorwasserstoffentwicklung (wird über einen Kühler entspannt) wird abgekühlt, der Fluorwasserstoff abdestiliert und der Rückstand auf Eis ausgetragen. Nach Phasentrennung wird das organische Produkt getrocknet und destilliert.

Man erhält 665 g 2,3-Dichlor-2,3-difluor-1,4-benzodioxan vom Siedepunkt 82 bis 83°C/18 mbar.

### Beispiel 2

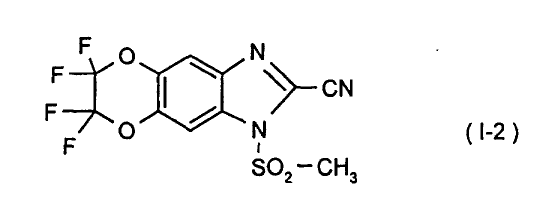

Zu 5,4 g (20 mmol) 2-Cyano-6,6,7,7-tetrafluor [1,4]dioxino[2,3-f]benzimidazol in 100 ml Acetonitril gibt man bei Raumtemperatur 5,4 g (40 mmol) pulverisiertes Kaliumcarbonat, tropft dann bei Raumtemperatur unter Rühren 4,6 g (30 mmol) Methansulfonsäurechlorid zu und erhitzt nach beendeter Zugabe für 6 Stunden auf Rückflusstemperatur. Anschließend wird die Reaktionsmischung auf Raumtemperatur abgekühlt und in 500 ml Wasser gegossen. Das Gemisch wird dreimal mit je 150 ml Methylenchlorid extrahiert. Die vereinigten organischen Phasen werden über Natriumsulfat getrocknet und unter vermindertem Druck vom Lösungsmittel befreit.

Man erhält 5,5 g (79 % der Theorie) 2-Cyano-3-methylsulfonyl-6,6,7,7-tetrafluor-[1,4]dioxino[2,3-f]benzimidazol vom Schmelzpunkt 180 bis 183°C.

Analog den Beispielen 1 und 2 sowie entsprechend der allgemeinen Beschreibung des erfindungsgemäßen Verfahrens werden die in der nachstehenden Tabelle aufgeführten Verbindungen der Formel hergestellt.

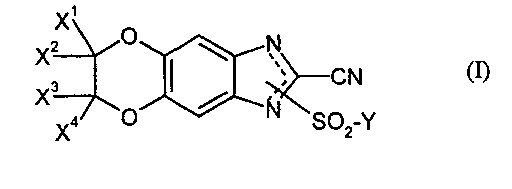

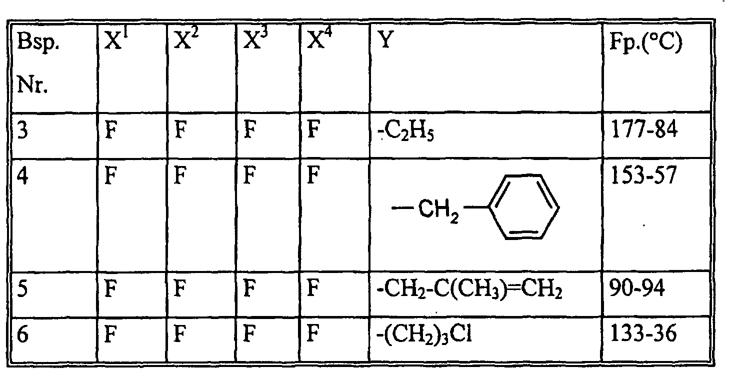

### Anwendungsbeispiele

In den folgenden Anwendungsbeispielen wurden die nachstehend aufgeführten Verbindungen als Vergleichssubstanzen eingesetzt. 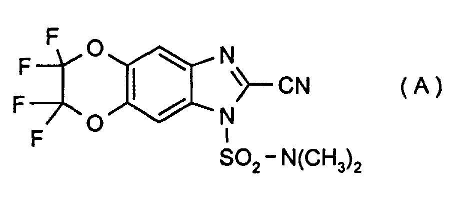
2-Cyano-3-dimethylaminosulfonyl-6,6,7,7-tetrafluor-[1,4]dioxino[2,3-f]benzimidazol 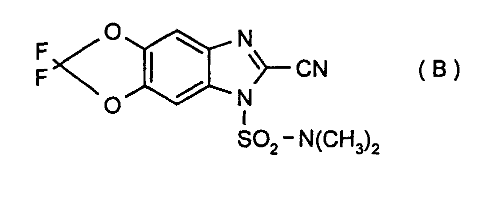
2-Cyano-6,6-difluor-3-dimethylaminosulfonyl-[1,3]dioxolo[4,5-f]benzimidazol 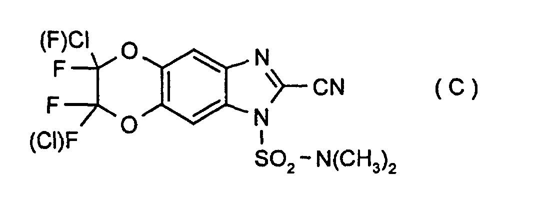
2-Cyano-3-dimethylaminosulfonyl-6,6,7-trifluor-7-chlor (bzw. 6,7,7-trifluor-6-chlor)-[1,4]-dioxino-[2,3-f]-benzimidazol
(Alle bekannt aus EP-A 0 517 476 bzw. DE-OS 4 139 950.)

### Beisniel A

### Venturia-Test (Apfel) / protektiv

| | | |
|---|---|---|
| Lösungsmittel | 4,7 | Gewichtsteile Aceton |
| Emulgator | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers Venturia inaequalis inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigen die Verbindungen der Formeln (I-1) und (I-2) bei einer Wirkstoffkonzentration von 10 ppm einen Wirkungsgrad von über 80 %, während die Vergleichssubstanzen (A), (B) und (C) einen Wirkungsgrad von 43 %, 0 % bzw. 18 % aufweisen.

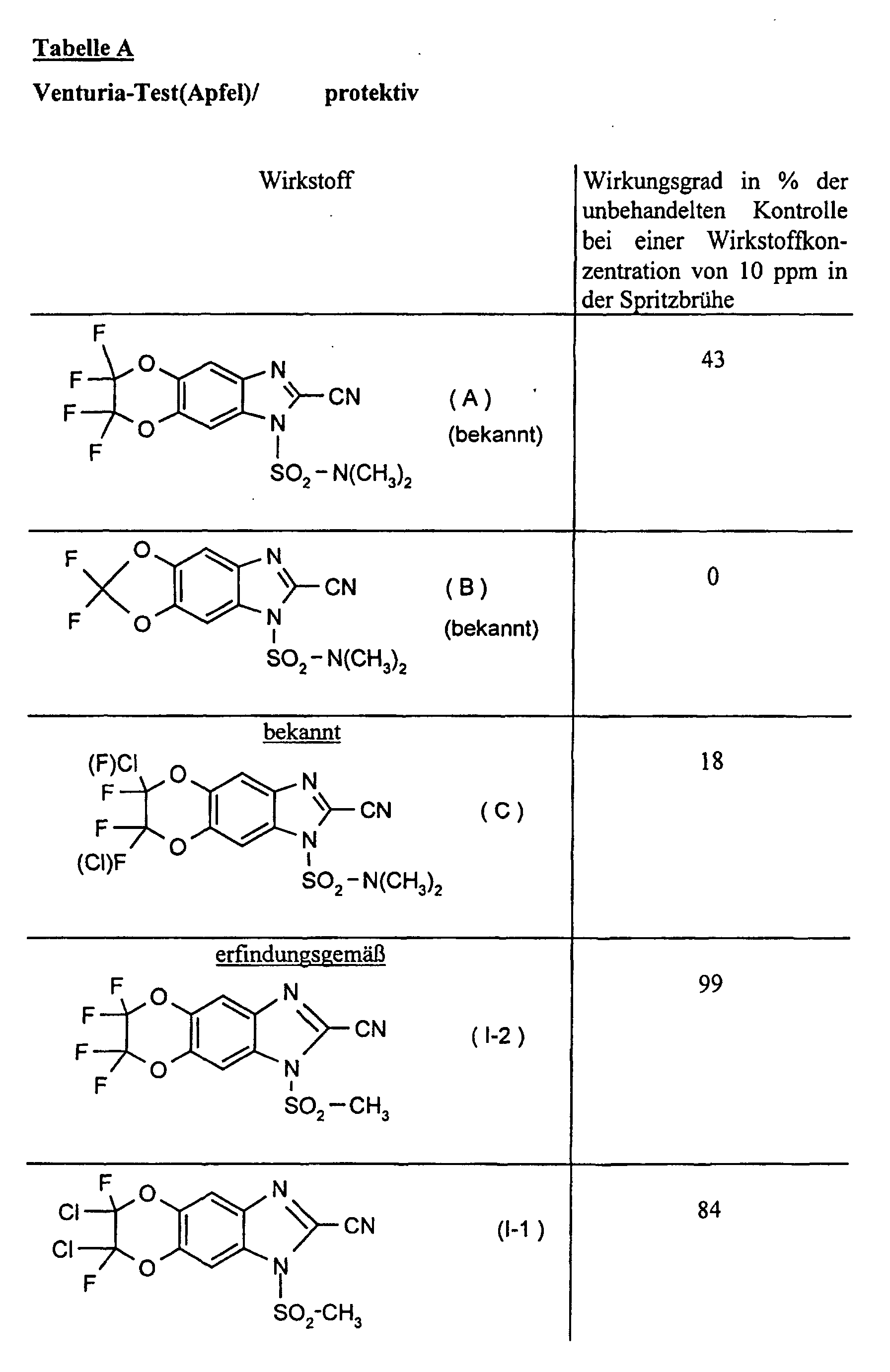

### Beispiel B

### Pyricularia-Test (Reis) / protektiv

| | | |
|---|---|---|
| Lösungsmittel | 12,5 | Gewichtsteile Aceton |
| Emulgator | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach Antrocknen des Spritzbelages werden die Pflanzen mit einer wässrigen Sporensuspension von Pyricularia oryzae inokuliert. Anschließend werden dei Pflanzen in einem Gewächshaus bei 100 % relativer Luftfeuchtigkeit und 25°C aufgestellt.

4 Tage nach der Inokulation erfolgt die Auswertung des Krankheitsbefalls.

In diesem Test zeigt die Verbindung der Formel (I-2) bei einer Wirkstoffkonzentration von 0,025 % einen Wirkungsgrad von 90 %, während die Vergleichssubstanz (A) einen Wirkungsgrad von 10 % aufweist.

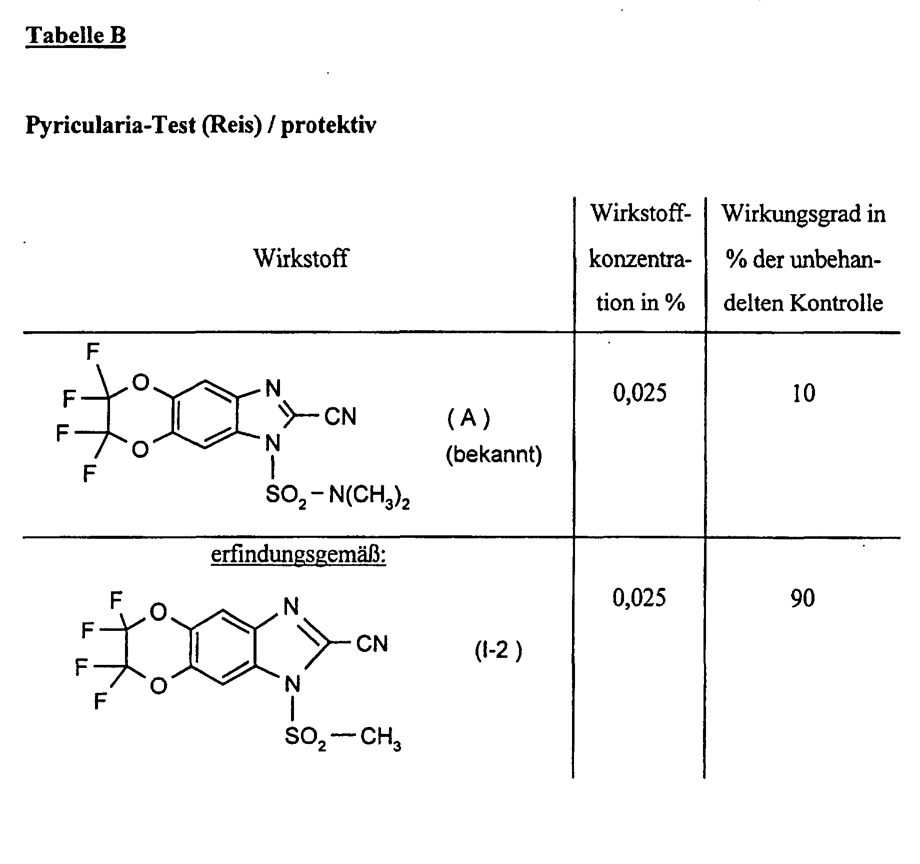

### Beispiel C

### Tetranychus-Test (OP-resistent / Tauchbehandlung)

| | | |
|---|---|---|
| Lösungsmittel | 7 | Gewichtsteile Aceton |
| Emulgator | 1 | Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Stadien der gemeinen Spinnmilbe Tetranychus urticae befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

In diesem Test zeigt die Verbindung der Formel (I-2) bei einer Wirkstoffkonzentration von 0,001 % nach 13 Tagen einen Abtötungsgrad von über 90 %, während die Vergleichssubstanz (A) einen Abtötungsgrad von 60 % bewirkt.

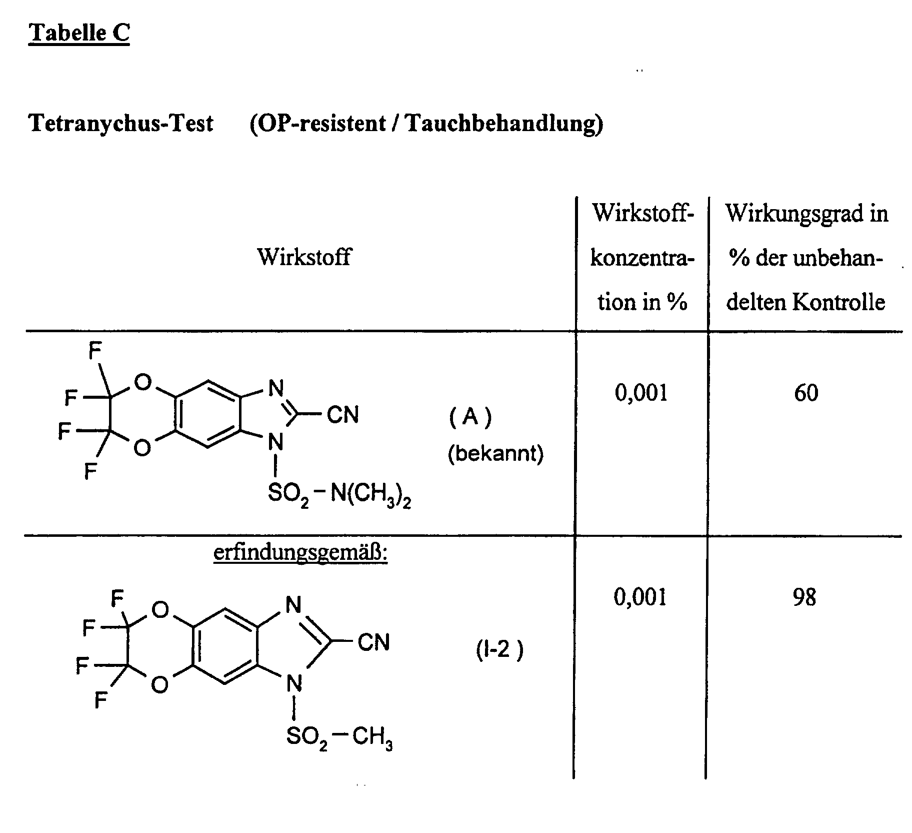

## Patentansprüche

1. Benzimidazol-Derivate der Formel 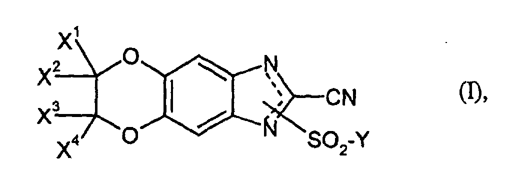 in welcher
X¹ und X³ für Fluor stehen,
X² und X⁴ für Chlor stehen und
Y für Methyl steht,
oder
X¹, X², X³ und X⁴ für Fluor stehen und
Y für Methyl, Ethyl, 3-Chlor-propyl, 1-Butenyl oder Benzyl steht.

2. Benzimidazol-Derivat gemäß Anspruch 1, **gekennzeichnet durch** die Formel

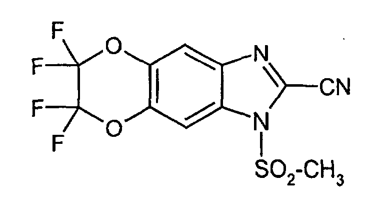

3. Benzimidazol-Derivat gemäß Anspruch 1, **gekennzeichnet durch** die Formel

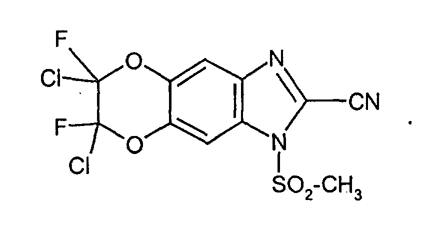

4. Verfahren zur Herstellung von Benzimidazol-Derivaten der Formel (I) gemäß Anspruch 1, **dadurch gekennzeichnet**, das man Benzimidazole der Formel 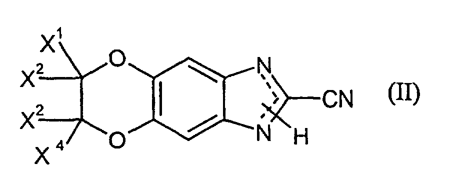 in welcher
X¹, X², X³ und X⁴ die oben angegebene Bedeutung haben,
mit Sulfonylchloriden der Formel
Cl-SO₂-Y (III)
in welcher
Y die oben angegebene Bedeutung hat,
gegebenenfalls in Gegenwart eines Verdünnungsmittels und gegebenenfalls in Gegenwart eines Reaktionshilfsmittels umsetzt.

5. Schädlingsbekämpfungsmittel, **gekennzeichnet durch** einen Gehalt an mindestens einem Benzimidazol-Derivat der Formel (I) gemäß Anspruch 1 neben Streckmitteln und/oder oberflächenaktiven Stoffen.

6. Verwendung von Benzimidazol-Derivaten der Formel (I) gemäß Anspruch 1 zur Bekämpfung von Schädlingen.

7. Verfahren zur Bekämpfung von Schädlingen, **dadurch gekennzeichnet, dass** man Benzimidazol-Derivate der Formel (I) gemäß Anspruch 1 auf die Schädlinge und/oder deren Lebensraum ausbringt.

8. Verfahren zur Herstellung von Schädlingsbekämpfungsmitteln, **dadurch gekennzeichnet, dass** man Benzimidazol-Derivate der Formel (I) gemäß Anspruch 1 mit Streckmitteln und/oder oberflächenaktiven Stoffen vermischt.

## Claims

1. Benzimidazole derivatives of the formula 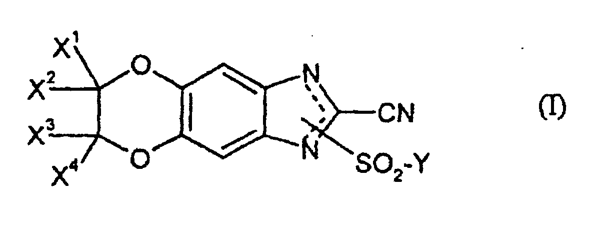 where
X¹ and X³ are fluorine,
X² and X⁴ are chlorine and
Y is methyl,
or
X¹, X², X³ and X⁴ are fluorine and
Y is methyl, ethyl, 3-chloro-propyl, isobutenyl or benzyl.

2. Benzimidazole derivative according to Claim 1, **characterized by** the formula

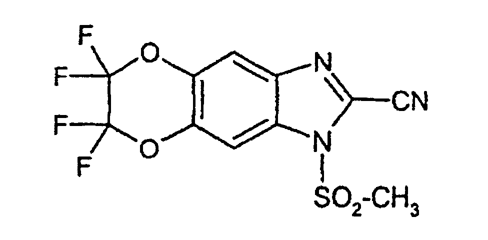

3. Benzimidazole derivative according to Claim 1, **characterized by** the formula

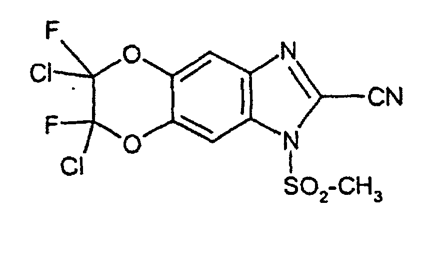

4. Process for the preparation of benzimidazole derivatives of the formula (I) according to Claim 1, **characterized in that** benzimidazoles of the formula 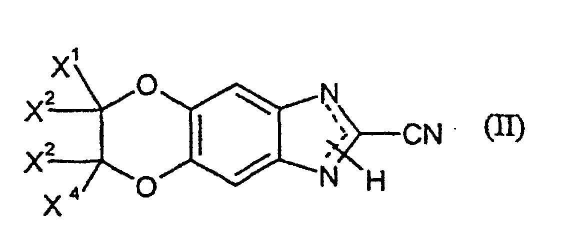 where
X¹, X², X³ and X⁴ have the abovementioned meanings
are reacted with sulphonyl chlorides of the formula
Cl-SO₂-Y (III)
in which
Y has the abovementioned meaning,
if appropriate in the presence of a diluent and if appropriate in the presence of a reaction auxiliary.

5. Pesticides, **characterized in that** they comprise at least one benzimidazole derivative of the formula (I) according to Claim 1 in addition to extenders and/or surfactants.

6. Use of benzimidazole derivatives of the formula (I) according to Claim 1 for controlling pests.

7. Method of controlling pests, **characterized in that** benzimidazole derivatives of the formula (I) according to Claim 1 are applied to the pests and/or their environment.

8. Process for the preparation of pesticides, **characterized in that** benzimidazole derivatives of the formula (I) according to Claim 1 are mixed with extenders and/or surfactants.

## Revendications

1. Dérivés de benzimidazole de formule 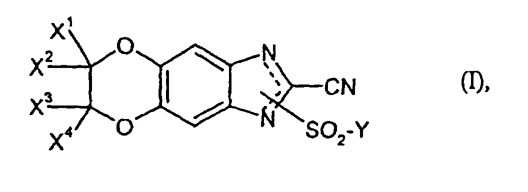 dans laquelle
X¹ et X³ représentent le fluor,
X² et X⁴ représentent le chlore et
Y est un reste méthyle,
ou bien
X¹, X², X³ et X⁴ représentent le fluor et
Y est un reste méthyle, éthyle, 3-chloropropyle, isobutényle ou benzyle.

2. Dérivé de benzimidazole suivant la revendication 1, **caractérisé par** la formule

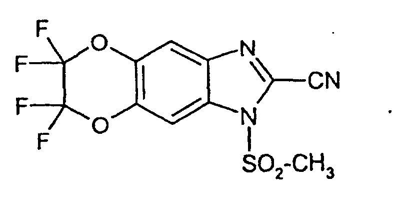

3. Dérivé de benzimidazole suivant la revendication 1, **caractérisé par** la formule

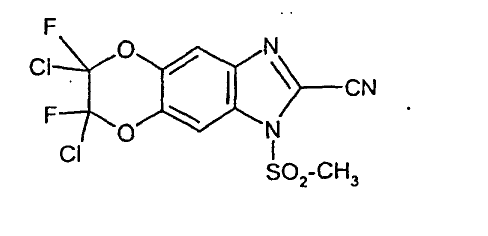

4. Procédé de production de dérivés de benzimidazole de formule (I) suivant la revendication 1, **caractérisé en ce qu'**on fait réagir des benzimidazoles de formule 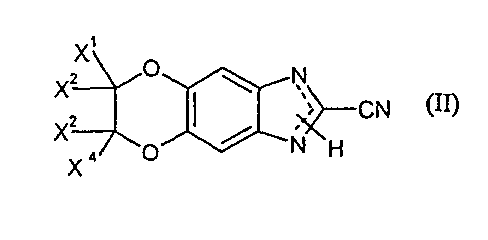 dans laquelle
X¹, X², X³ et X⁴ ont la définition indiquée ci-dessus,
avec des chlorures de sulfonyle de formule
Cl-SO₂-Y (III)
dans laquelle
Y a la définition indiquée ci-dessus,
éventuellement en présence d'un diluant et, le cas échéant, en présence d'un auxiliaire de réaction.

5. Compositions pesticides, **caractérisées par** une teneur en au moins un dérivé de benzimidazole de formule (I) suivant la revendication 1, à côté de diluants et/ou de substances tensio-actives.

6. Utilisation de dérivés de benzimidazole de formule (I) suivant la revendication 1 pour combattre des parasites.

7. Procédé pour combattre des parasites, **caractérisé en ce qu'**on épand des dérivés de benzimidazole de formule (I) suivant la revendication 1 sur les parasites et/ou sur leur milieu.

8. Procédé de préparation de compositions pesticides, **caractérisé en ce qu'**on mélange des dérivés de benzimidazole de formule (I) suivant la revendication 1 avec des diluants et/ou des agents tensio-actifs.
